Europäisches Patentamt

(19) European Patent Office

Office européen des brevets

(11) Publication number: **0 284 543**
**A2**

(12) **EUROPEAN PATENT APPLICATION**

(21) Application number: 88630054.0

(22) Date of filing: 24.03.88

(51) Int. Cl.4: **A 61 M 16/00**

(30) Priority: 24.03.87 IL 81986

(43) Date of publication of application:
28.09.88 Bulletin 88/39

(84) Designated Contracting States:
AT BE CH DE ES FR GB GR IT LI LU NL SE

(71) Applicant: TAMIR TEUS SCIENCE & MEDICAL
PRODUCTS LTD,
6 HaMagshimim Street
Petach Tikva (IL)

(72) Inventor: Gassner, Tsvi
37 Jabotinsky Street
Jerusalem (IL)

Weintraub, David
28 Harduf Street
Neve Ilan, Yavne (IL)

(74) Representative: Waxweiler, Jean et al
OFFICE DENNEMEYER S.à.r.l. P.O. Box 1502
L-1015 Luxembourg (LU)

(54) **Pressure controlled portable respirator.**

(57) A system for artificial respiration comprising a first gas supply line (22) leading from a first source of pressurized gas; a second gas supply line (24) leading from a second source of pressurized gas; and apparatus (20) connected to the first gas supply line (22) for providing pulses of gas to a patient at intervals, including apparatus for maintaining the pressure intensity of a pulse substantially constant throughout the duration thereof and also including pressure regulated control apparatus for governing the pressure intensity and the duration of the pulse and the intervals, the control apparatus being connected to the second gas supply line (24) and being operated by gas supplied therethrough.

FIG 1

## Description

The present invention relates to respirators in general and in particular to portable pressure controlled respirators.

In the field of artificial respiration, a number of devices are known. These devices include generally non-portable machines which comprise, in their simplest form, a source of gas, a gas flow path leading to an outlet, a gas flow control installed in the flow path, and an outlet, which may comprise a mask that is attached around the nose and mouth of a patient such that the gas is fed directly from the outlet to the patient's respiratory system.

A particular principle of artificial respiration is that a desired gas or a mixture of a number of gases is momentarily forced into the patient's lungs and then stopped, then the gas is once again forced in and again stopped, thereby simulating a respiratory cycle, which cycle is repeatedly applied to the patient in an attempt to stimulate the independent operation of the patient's respiratory system.

More recently, a number of patient valves' have been developed to automatically govern the artificial respiratory cycle, these generally defining a gas flow path through which gas is periodically conducted to the patient.

Disclosed in U.S. Patent No. 4,004,603 is a gas valve mechanism especially suitable for use as the patient valve in conjunction with a lung ventilator or a resuscitation device by developing high actuating forces from low pressure gas supplies. It comprises a valve element movable in a body having a gas pulse inlet, a gas pulse outlet and an exhaust outlet. The valve element has a large effective area exposed to the gas pulse inlet and is moved by gas pressure at that inlet, against spring-loading, to open a restricted flow path to the gas pulse outlet, the flow path restriction developing a pressure differential acting to oppose the spring loading and hold the valve element positively in the flow path open position.

It is an aim of the present invention to provide an automatic, pressure controlled, portable respirator that comprises a compact respiratory unit utilizing a gas source, which unit controls the timing of the respiratory cycle and also supplies constant pressure intensity gas pulses to the patient at a fixed expirium-inspirium ratio of two thirds to one third of a respiratory cycle, the duration of which is variable.

There is thus provided in accordance with the invention, a system for artificial respiration, comprising a first gas supply line leading from a first source of pressurized gas, a second gas supply line leading from a second source of pressurized gas, and apparatus connected to the first gas supply line for providing pulses of gas to a patient at intervals, and including apparatus for maintaining the pressure intensity of a pulse substantially constant throughout the duration thereof, and including pressure regulated control apparatus for governing the pulse and the intervals, which control apparatus is connected to and operated by gas supplied by the second gas supply line.

Additionally in accordance with the invention, the apparatus for providing comprises a housing, a pressure responsive valve element located within the housing, defining first and second ends and for operation in first and second modes, the first mode corresponding to the inspirium portion of a respiratory cycle and the second mode corresponding to the expirium portion of a respiratory cycle, and conduit apparatus defining a gas flow path extending from the second gas supply line connection with the control apparatus, through the control apparatus and terminating at a location corresponding to the first end of the valve element, for permitting the passage of pulses of pressurized gas to the valve element, provision of a pulse to the first end thereof corresponding to the first mode of operation, and an interval between pulses corresponding to the second mode of operation.

Further in accordance with the invention, the pressure responsive valve element comprises a cylinder having first and second ends and includes a gas inlet port for connection with the first gas supply line, a gas outlet port for supply of the gas to a patient, and an exhaust outlet, and a spring loaded piston, mounted within the cylinder and adapted for reciprocating axial movement between first and second end positions within the cylinder, movement towards the second end of the cylinder, which opposes the spring loading, being caused by the supply of a pulse of pressurized gas to the first end of the cylinder and corresponding to the first mode of operation, the movement towards the second end permitting pressurized gas supplied to the gas inlet port to flow past the second end of the piston and out through the gas outlet port, and movement towards the first end of the cylinder, in the direction of the spring loading and corresponding to the second mode of operation, being operative to obstruct the flow of gas past the piston and out through the gas outlet port, thereby permitting the passage of any air expelled from the respiratory system of a patient along a flow path extending from the gas outlet port, past the piston and through the exhaust outlet into the atmosphere.

Additionally in accordance with the invention, and wherein there is also provided pressure regulated adjustable control apparatus for providing pulses of pressurized gas to the first end of the cylinder, the provision of a pulse comprising the inspirium portion of a respiratory cycle and an interval between pulses comprising the expirium portion of the respiratory cycle, wherein the inspirium endures for one third part of the respiratory cycle and the expirium endures for two thirds of the respiratory cycle.

The present invention will be more fully understood and appreciated from the detailed description of a preferred embodiment, taken in conjunction with the drawings in which:

Fig. 1 is a schematic illustration of apparatus for artificial respiration, designed and constructed in accordance with a preferred embodiment of the present invention;

Fig. 2 is a side elevation of a respiratory unit, designed and constructed in accordance with a preferred embodiment of the present invention;

Fig. 3 is a plan view of the respiratory unit shown in Fig. 2;

Fig. 4 is an end elevation of the respiratory unit shown in Fig. 2;

Fig. 5 is a partially cut away side view of the respiratory unit of the present invention, corresponding to Fig. 2;

Fig. 6 is a partially cut away plan view of the respiratory unit of the present invention, corresponding to Fig. 3;

Fig. 7 is a partially cut away end view of the respiratory unit of the present invention, corresponding to Fig. 4; and

Figs. 8A and 8B are sectional views taken along line VIII-VIII in Fig. 2, showing the respiratory unit of the present invention in inspirium and expirium modes of a respiratory cycle respectively.

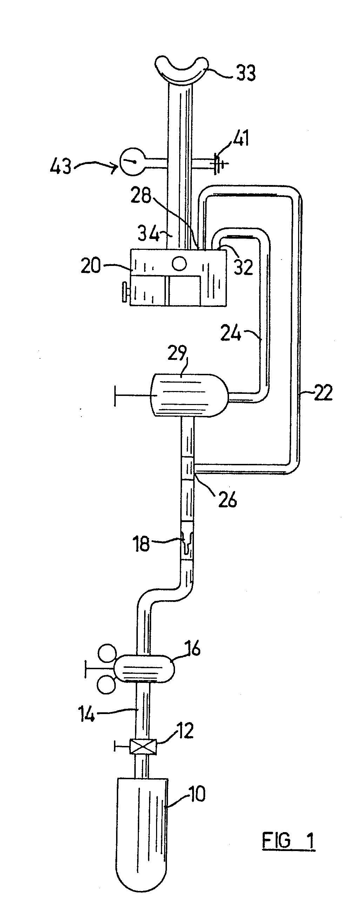

Referring to Fig. 1, there is shown in schematic form apparatus for artificial respiration according to the present invention. At reference numeral 10 there is depicted a gas cylinder, which typically contains air or oxygen at a pressure of 150-200 atmospheres, and which may be opened or closed by means of a gas tap 12.

Installed in the gas line 14, leading from gas cylinder 10 is a valve 16 which restricts the pressure of the gas exiting therefrom to a fixed maximum level, regardless of the pressure level of the gas entering thereinto. A typical valve used in association with gas cylinders limits the pressure to 4 atmospheres, although a lower level than this will suffice in connection with the present invention. Downstream of valve 16 is a connection 18 to a gas tap associated with a central gas supply, such as is found in hospitals, and which may be used in preference to a gas cylinder where appropriate.

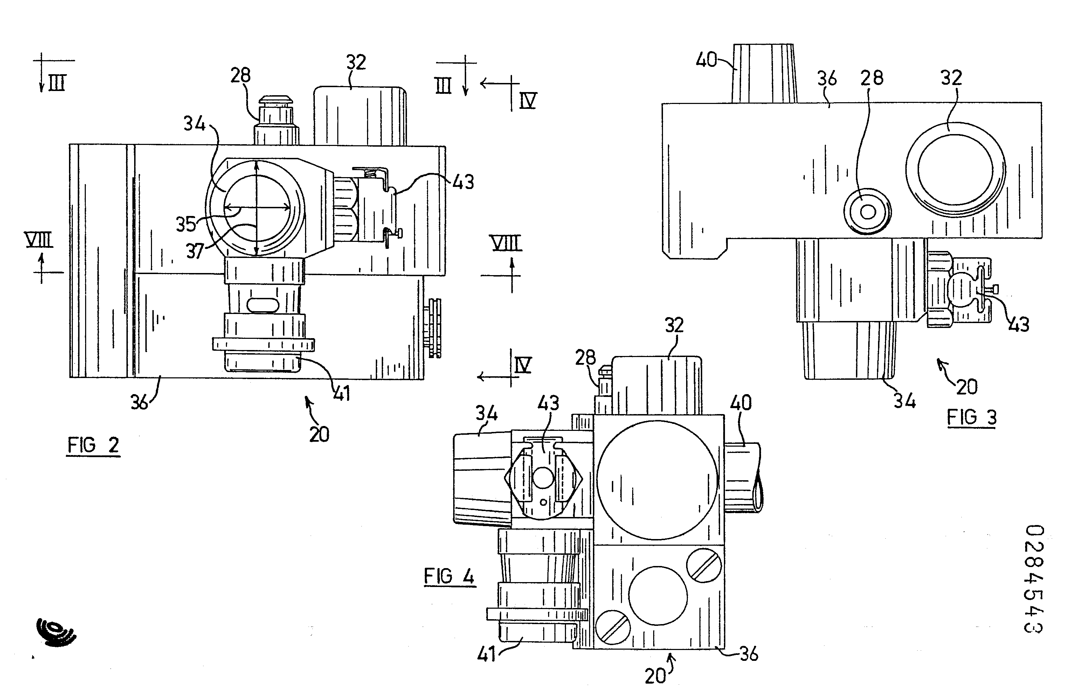

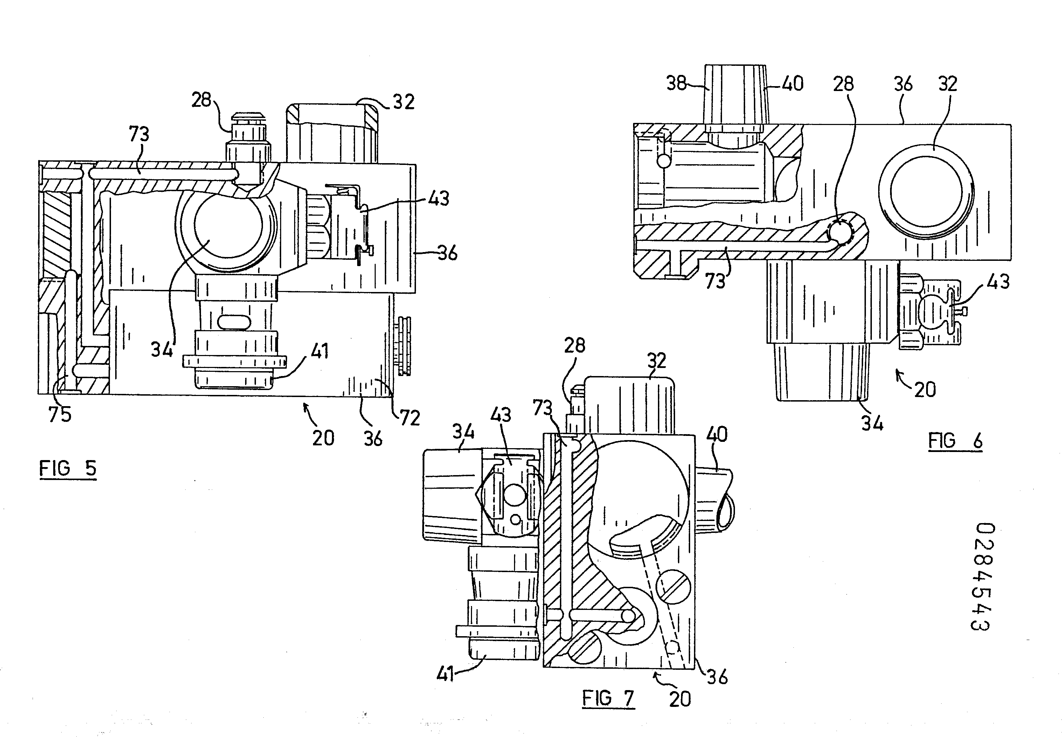

Shown by reference numeral 20 is a patient valve or respiratory unit, designed and constructed in accordance with a preferred embodiment of the present invention, which unit will be described in more detail below in conjunction with Figs. 2 to 8B.

Extending between gas line 14 and respiratory unit 20 are first and second gas conduits 22 and 24 respectively. First conduit 22 defines a gas flow path between a connection point 26 in gas line 14 and a gas entry point 28 located on respiratory unit 20, and second conduit 24 defines a gas flow path between a variable, in-line gas valve 29 and a gas entry port 32 located on the respiratory unit.

It is a particular feature of the invention that two gas supply lines are provided, as described above, thereby supplying to respiratory unit 20 two sources of gas that are independent of each other. This, as will be explained in more detail below, enables the provision to a patient of a gas pulse at a pressure that is maintained substantially constant throughout its duration.

It will be obvious to persons skilled in the art that although a single gas source supplied by way of gas line 14 has been shown and described in accordance with a preferred embodiment of the invention, this need not be so. Indeed, in an alternative embodiment of the invention, first and second gas conduits, 22 and 24 respectively, may be supplied from two independent sources.

Now, with additional reference to Figs. 2 to 8B, respiratory unit 20 and the operation thereof will be described. Respiratory unit 20 comprises a housing 36, in which there is provided a cylinder 38. Housing 36 comprises gas entry point 28 and gas entry port 32, and further comprises a gas outlet port 34 for supply of gas to a patient, and an exhaust outlet, denoted by reference numeral 40, and the purpose of which will be explained below.

Now, with particular reference to Fig. 2, gas outlet port 34 is shown to define inner and outer diameters, 35 and 37 respectively. Outer diameter 37 is configured to be connectable with a respiratory face mask, schematically shown at 33 in Fig. 1, which mask is configured for retention over the nose and mouth' of a patient, while inner diameter 35 is configured to be connectable with a tracheal tube, (not shown), wherein one end of the tube is connectable with gas outlet port 34 and a distal end thereof is configured for insertion into the trachea of a patient.

Located adjacent gas outlet port 34 is a safety relief valve 41 which, in case of a build up of excess pressure, is adapted to allow the outflow of sufficient pressurized gas that will prevent the gas supply to the patient being supplied at a pressure that is too high and which may thus be dangerous.

There is further provided, at location 43, a pressure gauge, for regulation of the pressure level of gas supplied to a patient.

Retained within cylinder 38 is a spring loaded piston 42 which is adapted for reciprocating axial movement between first and second end positions within cylinder 38, the first and second ends of the cylinder being referenced 44 and 46 respectively.

Movement towards second end 46 of cylinder 38 opposing the spring loading, which movement is caused by a supply of pressurized gas to first end 44 thereof and corresponds to a first mode of operation, is operative to permit pressurized gas supplied to gas entry port 32 to flow past a second end 48 of piston 42 and out through gas outlet port 34.

However, movement of piston 34 in the opposite direction to that described, in the direction of the spring loading and corresponding to a second mode of operation, is operative to prevent the flow of gas past piston 42, rather, a flow path extending from gas outlet port 34, past piston 42 and through exhaust outlet 40 into the atmosphere permits flow therealong of any air expelled from the respiratory system of the patient.

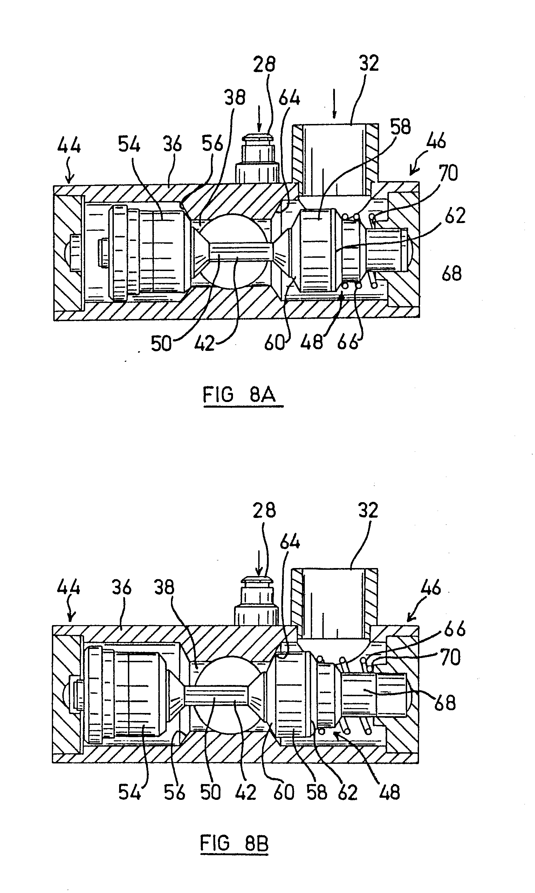

As shown particularly in Figs. 8A and 8B, piston 42 comprises a piston rod 50, the diameter of which is significantly smaller than the internal diameter of cylinder 38. This facilitates the passage of air expelled by the patient along a flow path extending from gas outlet port 34, past piston rod 50 and through exhaust outlet 40.

Mounted on piston rod 50 and denoted by reference numeral 54 is a first end engagement element which in the first mode of operation is brought into contact with an annular surface 56 formed within cylinder 38.

Also mounted on piston rod 50 is a second end spigot element 58 which has first and second faces, 60 and 62 respectively. First face 60 is configured such that in the second mode of operation it sealingly engages an annular surface 64 thereby obstructing the passage of gas along the first mode gas flow path extending therepast.

A compression spring 66 is coaxially mounted with a second face protrusion 68, and is fixed between second face 62 of spigot element 58 and an inner face 70 of second end 46 of cylinder 38.

Also contained within housing 36 is a pressure regulated control device shown schematically at 72. Control device 72 is operated by a supply of gas supplied at gas inlet point 28. Gas at pressure passes from gas inlet point 28, through gas conduit 73 and into control device 72.

Control device 72 may be preset to provide a preferred number of respiratory cycles per minute, typically ranging from 8 to 100 cycles per minute, wherein a complete cycle comprises inspirium, or forcing gas into the respiratory system of a patient, and expirium, permitting the patient to breath out if he is able.

In a preferred embodiment of the invention, the ratio of inspirium to expirium is approximately one third of a cycle to two thirds of a cycle, as this has been medically determined to be the ratio most likely to stimulate the patient's own respiratory system.

Although control device 72 is a standard product, for example, the ADJUSTABLE IMPULSE GENERATOR, model no. 81 507 6, as manufactured by CROUZET S.A., 25 Rue Jules Vedrines, BP 1014, Valence, Cedex, France, and is therefore not discussed here in detail, its function is to periodically supply pulses of pressurized gas to first end 44 of cylinder 38 via a gas conduit 75. The build up of pressure on piston 42 forces it to move axially in a second end direction, thereby compressing spring 66 and at the same time opening the first mode gas flow path between gas entry port 30 and gas outlet port 34.

At the end of each pulse generated by control device 72, pressure is released from piston 42 and spring 66, in regaining its non-compressed configuration returns the piston to a first end position, thereby causing the obstruction of first mode gas flow path by spigot element 58. It will be obvious that the time duration of each pulse is equivalent to the time duration of the inspirium, and that the time interval between each pulse is equivalent to the time duration of the expirium.

It will be appreciated by persons skilled in the art, that the ability to provide a gas pulse having a pressure that is substantially constant throughout its duration, as mentioned above, is due to the provision of two independent gas sources to respiratory unit 20; gas supplied at gas entry port 32 is supplied directly from a gas source, and is supplied to the patient when the gas flow extending from gas entry port 32 to gas exit port 34 is not blocked by piston 42. The movement of piston 42 is independently governed by the supply of gas at gas entry point 28.

It is an important feature of the invention that the pressurized gas supply passing through second gas conduit 24 and which passes on to the patient, is subject to a pressure reduction imposed by variable in-line gas valve 29. The importance of this is obvious when giving regard to the range of maximum gas pressures applied in a typical pulse to a patient, which maximum range typically varies from 0.02 atmospheres for a child to 0.06 atmospheres for an adult, while the pressure of the gas in line 14 upstream of valve 29 is typically 4 atmospheres.

It will be obvious to persons skilled in the art that the present invention is not limited to what has been shown and described hereinabove. Rather, the scope of the present invention is limited solely by the claims which follow:

**Claims**

1. A system for artificial respiration, comprising:
   a first gas supply line leading from a first source of pressurized gas;
   a second gas supply line leading from a second source of pressurized gas; and
   means connected to said first gas supply line for providing pulses of gas to a patient at intervals, including means for maintaining the pressure intensity of a pulse substantially constant throughout the duration thereof, and also including pressure regulated control means for governing the pressure intensity and the duration of said pulse and said intervals, said control means being connected to said second gas supply line and also being operated by gas supplied therethrough.

2. A system for artificial respiration according to claim 1, and wherein said means for providing comprises:
   a housing;
   a pressure responsive valve element located within said housing for operation in first and second modes, said first mode corresponding to the inspirium portion of a respiratory cycle and said second mode corresponding to the expirium portion of a respiratory cycle; and
   conduit means defining a gas flow path extending from said second gas supply line connection with said control means, through said control means and terminating adjacent said valve element, for permitting the supply of pulses of pressurized gas adjacent said valve element, generation of each of the pulses corresponding to said first mode of operation, and an interval between said pulses corresponding to said second mode of operation.

3. A system for artificial respiration according to claim 2, and wherein said pressure responsive valve element comprises:

a cylinder having first and second ends and including a gas inlet port for connection with said first gas supply line, a gas outlet port for supply of said gas to a patient, and an exhaust outlet; and

a spring loaded piston mounted within said cylinder for reciprocating axial movement between first and second end positions within said cylinder, movement towards said second end of said cylinder opposing said spring loading and being caused by provision of a pulse of gas to said first end of said cylinder and corresponding to said first mode of operation, said movement towards said second end permitting pressurized gas supplied to said gas inlet port to flow past said second end of said piston and out through said gas outlet port, while movement towards said first end of said cylinder in the direction of said spring loading and corresponding to said second mode of operation, is operative to obstruct said flow of gas past piston and out through said gas outlet port, thus permitting the passage of air expelled from the respiratory system of a patient along a flow path extending from said gas outlet port, past said piston and through said exhaust outlet into the atmosphere.

4. A system for artificial respiration according to claim 3, and wherein said spring loaded piston comprises:

a piston rod, having first and second ends corresponding to said first and second ends of said cylinder and axially located therewithin, said piston rod having a diameter less than the internal diameter of said cylinder, said second mode gas flow path extending from said gas outlet, past said piston rod in a direction transverse thereto, and through said exhaust outlet;

an engagement element mounted on said piston rod adjacent said first end thereof, which is configured such that in said first mode of operation it is brought into contact with a first annular surface formed within said cylinder;

a spigot element having first and second faces, mounted by said first face thereof on said piston rod adjacent said second end of said rod, said first face being configured such that in said second mode of operation said element engages a second annular surface formed within said cylinder and thereby obstructs the passage of gas along said first mode gas flow path extending therepast; and

a compression spring, coaxially mounted with a second face axial protrusion, and fixed between said second face of said spigot element and said second end of said cylinder.

5. A system for artificial respiration according to claim 4, and wherein said pressure regulated control means comprises adjustable control means for supplying a pulse of gas to said first end of said cylinder, and wherein supply of said pulse corresponds to the inspirium portion of a respiratory cycle and an interval between pulses corresponds to the expirium portion of the respiratory cycle, the inspirium enduring for one third part of the respiratory cycle and the expirium enduring for two thirds of the respiratory cycle.

6. A system for artificial respiration according to any of claims 3 to 5, and wherein said gas outlet port is connectable with:

a respiratory face mask, configured for retention over the nose and mouth of a patient; and

a tracheal tube, wherein one end of said tube includes means for connection to said gas outlet port and a distal end thereof is configured for insertion into the trachea of a patient.

7. A system for artificial respiration according to any of claims 3 to 6, and also comprising a pressure gauge at said gas outlet port.

8. A system for artificial respiration according to any of claims 3 to 7, and also comprising a safety relief valve located adjacent said gas outlet port.

9. A system for artificial respiration according to any of the preceding claims, and wherein said first gas supply line additionally comprises an adjustable valve for governing the pressure of gas supplied to a patient.

10. A system for artificial respiration according to any of the preceding claims, and also comprising a fixed pressure valve in said first and second gas supply lines for limiting the pressure of gas flowing in said lines.

11. For use in a system for artificial respiration comprising a source of pressurized gas and first and second gas supply lines, means connected to the first gas supply line for providing pulses of gas to a patient at intervals, said means for providing including pressure regulated control means connected to said second gas supply line for governing the pressure intensity and the duration of the pulses and the intervals.

12. Means for providing according to claim 11, and comprising:

a housing;

a pressure responsive valve element located within said housing, for operation in first and second modes, said first mode corresponding to the inspirium portion of a respiratory cycle and said second mode corresponding to the expirium portion of a respiratory cycle; and

conduit means defining a gas flow path extending from said second gas supply line connection with said control means, through said control means and terminating adjacent said valve element, for permitting the passage of pulses of gas to said valve element, generation of each of the pulses corresponding to said first mode of operation, and an interval between the pulses corresponding to said second mode of operation.

0284543

FIG 1

FIG 2

FIG 3

FIG 4

**FIG 5**

28    32

73    43

36

34    41

75    20    36    72

**FIG 6**

38   40   28   36   32

73    43

34    20

**FIG 7**

28   32

34   43   73   40

41    20   36

0284543

0284543

FIG 8A

FIG 8B